Europäisches Patentamt

European Patent Office

Office européen des brevets

(19)

(11) Publication number: **0 207 152 B1**

(12)

# EUROPEAN PATENT SPECIFICATION

(45) Date of publication of patent specification: **30.12.92**    (51) Int. Cl.⁵: **G01N 33/53**, G01N 33/543

(21) Application number: **86900694.0**

(22) Date of filing: **19.12.85**

(86) International application number:
**PCT/US85/02534**

(87) International publication number:
**WO 86/03839 (03.07.86 86/14)**

The file contains technical information submitted
after the application was filed and not included in
this specification

(54) **SOLID PHASE DIFFUSION ASSAY.**

<table>
<tr><td>

(30) Priority: **20.12.84 US 684059**
**02.08.85 US 761961**

(43) Date of publication of application:
**07.01.87 Bulletin 87/02**

(45) Publication of the grant of the patent:
**30.12.92 Bulletin 92/53**

(84) Designated Contracting States:
**AT BE CH DE FR GB IT LI LU NL SE**

(56) References cited:
**EP-A- 0 046 004      EP-A- 0 066 648**
**EP-A- 0 120 602      WO-A-79/00044**
**DE-A- 2 804 940      FR-A- 2 482 308**
**US-A- 4 435 504**

</td><td>

(73) Proprietor: **NYCOMED IMAGING AS**
**Nycoveien 2, Postboks 4220**
**Oslo 4(NO)**

(72) Inventor: **CERNY, Erich H.**
**100 West University Parkway**
**Baltimore, MD 21210(US)**

(74) Representative: **Holmes, Michael John et al**
**Frank B. Dehn & Co. Imperial House 15-19**
**Kingsway**
**London WC2B 6UZ(GB)**

</td></tr>
</table>

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

Rank Xerox (UK) Business Services

**Description**

This invention relates to the quantitative and qualitative assay of small amounts of substances in a solution and more particularly to the rapid and simple identification and quantification of substances in solution by a novel solid phase diffusion assay technique. The novel assay may be adapted to rapidly and quantitatively determine the concentration of proteins, hormones, drugs, polypeptides, vitamins, glycosides and the like. The present invention further relates to a kit for effecting such quantitative measurements and to certain novel components of such kits and for use in the novel assay.

There is a continuing need for an inexpensive, easy to perform method of detecting substances that are present in fluids at concentrations of the order of $1 \times 10^{-6}$ grams/ml or less. Prior art methods capable of accurately detecting a substance in a fluid at these concentrations are cumbersome, expensive and require long periods of time to perform. In addition, expensive and complicated equipment is required to perform these prior art methods.

There are many prior art assay methods designed to detect the presence of soluble substances in serum and other media of biological importance. For substances that have biological activity, one can simply measure the activity in the biological fluid to detect the presence of the substance. For example, one can measure the presence of the enzyme acid phosphatase in blood serum by adding substrate of acid phosphatase enzyme such a p-nitrophenyl phosphate and incubating the solution for a period of time. If the enzyme is present in the blood, the solution will turn yellow as the substrate is hydrolyzed by the enzyme to phosphate and p-nitrophenol. However, there are many problems associated with this type of assay. For example, the substance to be assayed must have a biological activity that can be measured. Often the measurement of biological activity can be cumbersome and very time consuming. Furthermore, the activity of the enzyme may be inhibited by the presence of an inhibitor. If such an inhibitor is present, a falsely low activity will be measured. In addition the enzyme may be present in the fluid but may be inactive.

Another method of measuring the presence of trace substances in biological fluids is chromatography. There are many different types of chromatography. Thin layer chromatography, in combination with mass spectroscopy or gas phase chromatography, has been used to isolate and quantify a particular substance in biological fluids. However, thin layer chromatography has a number of deficiencies such as being slow, being subject to a wide range of interfering materials, and suffering from severe fluctuations in reliability.

Liquid chromatography is another method of isolating materials from biological fluids. In this method, advantage is taken of particular molecule's physical properties, such as size or charge. However, one still must utilize a method of analyzing the particular substance after it is isolated. This can be done by measuring biological activity, absorbance characteristics, mass spectroscopy, or by further separation analysis.

Another method that takes advantage of molecular charge and size is gel electrophoresis. In this method, a biological sample is placed on a porous gel. The sample and the gel are then subjected to an electrical field causing the sample to migrate through the gel. The rate of migration is dependent upon the charge and on the size of the molecule. In this way, different molecules can be separated and isolated.

There are many problems with chromatographic and electrophoretic methods for identification of substances. One of the problems is in identifying the substance after it has been isolated. In order to identify the isolated substance, one must perform another procedure such as measurement of biological activity, analysis by mass spectroscopy or identification by other methods, such as immunological methods. An electrophoresis or chromatography procedure is a time consuming process taking several hours to several days. In addition, the equipment used in these procedures is expensive and requires an experienced technician to perform the analysis.

Another method of identifying trace amounts of a particular substance in a solution is through immunological techniques. All immunological procedures use an antigen, and an antibody which is specific for the antigen. Prior art immunological methods include immunological precipitation in which the antibody combines with an antigen for which the antibody is specific. The resulting complex precipitates out of solution forming a visible precipitate.

Agglutination is another prior art method of detecting small concentrations of a particular substance. In agglutination, a body, such as a red blood cell or a bacteria, is reacted with antibodies that are specific for an antigen on the surface of the body. As the antibodies react with the surface antigens, the cells agglutinate forming a dense, visible clump.

The procedures of immunoprecipitation and immunoagglutination suffer from a general lack of sensitivity. In addition, the procedures require the antigen to have multiple antibody binding sites so that the antibodies may crosslink the antigens causing the precipitation or agglutination. The process of immunoprecipitation requires several hours to several days to complete thereby making the procedure impractical for many situations where the identification or quantification of a particular substance must be performed quickly.

The problem of lack of precision by the above described procedures was overcome by the procedure known as radioimmuno assay. In this procedure, the antigen to be measured is "labeled" with a radioactive element to form a radioactive analogue. Radioactive isotopes that are commonly used in radioimmunoassays are shown in Table I.

TABLE I

| Radioactive Isotopes used for Tagging Biological Materials | | |
|---|---|---|
| Isotope | Specific Activity of Pure Isotope | |
| | (Curies per mole) | Half-life |
| $C^{14}$ | $6.25 \times 10^1$ | 5230 years |
| $H^3$ | $2.91 \times 10^4$ | 12.3 years |
| $S^{35}$ | $1.50 \times 10^6$ | 87 days |
| $I^{125}$ | $2.18 \times 10^6$ | 60 days |
| $P^{32}$ | $3.16 \times 10^6$ | 14.3 days |
| $I^{131}$ | $1.62 \times 10^7$ | 8.1 days |

By mixing an antibody with solutions of a hapten or antigen to be analyzed, and with the radioactive antigen analogue, the radioactive analogue will be prevented from binding to the antibody to an extent directly proportional to the concentration of the hapten or antigen in the solution. By then separating and assaying the free radioactive analogue from the antibody bound radioactive analogue, one can indirectly determine the amount of hapten or antigen in the original solution.

However, the use of radioisotopes in such an assay is a potential health hazard and, furthermore, the instrumentation required for radioimmunoassay is relatively sophisticated and expensive. Another problem with the radioimmunoassay is in labeling the antigen or antibody. The isotopes that are most commonly used are those with a short half-life. These include Iodine-131 and Iodine-125. Because these isotopes have such a short half-life (8.1 days and 60 days, respectively), the labeled component of an assay must periodically be replaced with new product. In addition, a standard curve must be prepared with each unknown sample since the specific activity of the isotope is constantly decreasing. A further problem with some labeled components is autodegradation. The isotopes that are commonly used to label the compounds are relatively strong radiation emitters and can cause the compounds to which they are attached to be degraded. Finally, with the advent of increasing number of government regulations concerning the disposal of radioactive wastes, disposing of the radioactive isotopes used in radioimmuno assays has become an increasingly difficult and expensive problem.

Enzyme immunoassays overcome the above problems and in addition, have the unique advantage of potential amplification of the measured activity. (The field of enzyme immunoassays has been extensively reviewed in Developments in Immunology, Vol. 18, Immunoenzymatic Techniques, Elsevier Science Publishers, 1983) This method replaces the radioactive biological substance analogue with an enzyme labeled biological substance (hapten or antigen). Typical enzymes that can be used as labels in the enzyme immunoassay are listed in Table II.

## Table II

| Enzymes commonly used in enzyme immuno assay |
|---|
| Alkaline phosphatases |
| Glucose oxidases |
| Ureases |
| Peroxidases |
| ß-Galactosidases |
| Glucose-6-phosphate dehydrogenases |
| Lysozymes |
| Malate dehydrogenases |

Such modified enzyme molecules retain their enzymatic activity and the enzyme-labeled biological substance will compete for antibody complex formation with the unknown amount of free biological substance in the system. The complexes may be separated in view of the insolubility in certain substances. The activity of the separated complex, or the part remaining in solution, is used as a measure of the amount of antigen originally present. The same principle may be applicable to a reverse system, using enzyme-labeled antibodies whenever the unmodified version of the same antibody present in biological fluids has to be determined.

There are several variations of the enzyme immuno assay. In one variation, known as enzyme-linked immunosorbent assay (ELISA), labeled and unlabeled antigen compete for attachment to a limited quantity of solid-phase antibody. The enzyme label that is displaced is measured, and the calculations that follow are essentially the same as in radioimmunoassay procedures.

The sandwich technique is another variation of enzyme immunoassay and relies on the multivalence of antigens and capacity to bind simultaneously with two molecules of antibody. The first antibody molecule is a solid phase reactant. It is used in excess to ensure binding of all the antigen molecules in the unknown sample. After that reaction is completed, an enzyme-labeled antibody is added and incubated with the complex resulting from the first phase. The labeled antibody then combines with available determinants on the antigen. Excess antibody is removed by washing and enzyme activity is then determined. As in other systems, the amount of enzyme bound to the complex is an indirect measure of the amount of antigen in the sample. Variations of this method include the second antibody method. In that method, antigen is reacted first with solid phase antibody and later with free antibody, neither of which is labeled. Then enzyme-labeled antibody with a specificity for the free antibody is used as the last reagent.

Most of the enzyme immunoassay techniques are classified as heterogeneous assays. This means that the bound labeled molecule must, at some point in the assay procedure, be separated from the free labeled molecule in order to perform the necessary calculations to determine the amount of unknown substance in the fluid. This requires a separation step in the assay and adds to both the time and expense of the assay procedure. There are enzyme immuno assay procedures that are homogeneous assays in that there is no separation of bound labeled substance and unbound labeled substance. Such a system does not require a solid phase reactant, but rather relies on an inhibition of enzyme activity by the combination of an antibody with an enzyme-labeled antigen or hapten. This type of assay is of limited usefulness since not all antigen-antibody combinations will result in a predictable diminution of enzyme activity.

Enzyme immunoassays are generally as sensitive as radioimmunoassays and are much safer because no radioactive isotopes are used. In addition, enzyme immunoassays generally require less sophisticated equipment than the radioimmunoassays. An enzyme immunoassay is generally much less expensive than a corresponding assay done by radioimmunoassay.

However, there are still significant problems associated with the typical enzyme immunoassay. The time required to run an enzyme immunoassay, for many applications, is too long. In most cases, an incubation period of at least several hours is required to perform the assay. In addition, the typical enzyme

4

immunoassay comprises several washing steps and an additional incubation step with an enzyme substrate to develop a color which can be measured. The color from the enzyme reaction must then be measured in a spectrophotometer.

A number of prior immunoassays have been proposed, as described hereinafter, which employ a receptor for the analyte immobilised on a porous support into which the analyte and other liquids are allowed to diffuse.

We have found that many of the disadvantages of the prior art diffusion-based assays may be overcome by using a gold or silver colloid as label, since this gives excellent sensitivity while avoiding the use of radioactive materials or enzymes. We have further found that the sensitivity of the assay is improved by application of the sample and other fluids through a hole in a cover sheet and/or by use of a hydrophilic layer which assists transverse diffusion of liquids through the porous support carrying the receptor substance.

European Patent Application 0007654 (Akzo), describes an immunoassay performed in wells of polystyrene microtiter plates in which the analyte is adsorbed onto the polystyrene surface through a first specific binding partner, and subsequently labelled with a second binding partner attached to a colloidal metal such as colloidal gold. The specifically attached metal colloid is preferentially liberated into a solution of hydrochloric acid and measured by photometric, or other analytical methods, or by the naked eye. However, there is no suggestion of application of a sample or a metal colloid-labelled binding partner through a hole in a cover layer, and/or to perform the reaction in the course of a rapid diffusion or filtration process in a porous medium. The analyte is, in fact, incubated with the activated support for periods of one hour to overnight, and the colloid-labelled binding partner is incubated routinely at 18 hours. This is in contrast to the very short reaction times achieved by the diffusion method of the invention.

European Patent Application 158746 describes a blot overlay assay wherein an analyte is adsorbed onto a blotting medium and labeled with a binding partner attached to a colloidal metal such as colloidal gold. However, there is no suggestion of application of a sample and/or a metal colloid-labelled binding partner to a support through a hole in a cover layer to give a precise origin of application and a possible concentration effect. The analyte is, in fact, incubated with the activated support for periods of 1 hour to overnight in contrast to the very short reaction times achieved by the diffusion method of the invention. There is no disclosure of the use of an absorbent layer to facilitate diffusion.

Analytical Biochemistry 142, 221-225 (1984) discloses a method of detection of antigens wherein an antibody is attached to nitrocellulose papers and a dot of applied sample applied, followed by incubation at 80°C for 1 hour. The paper is then incubated overnight at 4°C with antibody against the target antigen (rabbit serum), washed in four changes of PBS and then soaked in gold labeled anti-antibody. There is no disclosure of diffusion of the sample or other solutions and reaction times are lengthy. There is no disclosure of application of the solutions through a hole in a cover sheet or the use of an absorbent layer to enhance transverse diffusion.

DE 2804940 describes a diffusion based immunoassay (although not a radial diffusion assay) using an enzyme as label. There is no disclosure of the use of colloidal gold of the application of the sample and other fluids via a hole in a cover sheet.

EP 0046004 describes the adsorption of an analyte into a membrane having an adsorbent backing layer via a hole in a cover sheet but does not describe the use of a gold or silver colloid as a label.

FR 2482308 describes a linear diffusion immunoassay but does not describe the use of a gold or silver colloid as label, the application of the sample and other solutions via a hole in a cover sheet or the use of an adsorbent backing.

According to the present invention we provide a method for the quantitative or qualitative determination of an analyte in a liquid test sample comprising the steps of:

(a) binding a first substance to an insoluble porous sheet support, said first substance being selected from the group consisting of ligands and receptors binding said analyte, said support being covered by a layer of water-impermeable material having a hole therein and/or having adjacently beneath a sheet of hydrophilic material serving to enhance transverse diffusion of liquid through said support;

(b) applying said test sample to said insoluble support, through said hole, where said cover layer is present;

(c) permitting said test sample to diffuse through said insoluble support carrying said first substance;

(d) applying a labeled second substance to said insoluble support before, together with, or after application of said analyte, said second substance being selected from the group of ligands and receptors capable of being immobilized by binding to either said first substance or said analyte and being labeled with colloidal gold or colloidal silver; and

(e) assessing the presence and/or quantity of said immobilized labeled second substance.

For quantitative determination it is preferred that said label is present in an amount sufficient to provide a clear signal which is visible to the naked eye when said second substance is present in an amount above a predetermined detection limit, and which is not visible to the naked eye when said second substance is present in an amount below said predetermined detection limit.

The solid phase diffusion assay of the present invention is not a heterogeneous assay and therefore does not require a distinct separation step to separate bound labeled compounds from unbound labeled compounds. The present invention is free of many of the problems associated with the aforementioned methods of detecting substances present in the biological fluids in minute concentrations. It provides a solid phase diffusion assay which can be performed in a relatively short period of time and is comparable in sensitivity to the radioimmunoassay. In addition the solid phase diffusion assay of the present invention does not necessarily require any sophisticated measuring equipment. The solid phase diffusion assay of the present invention does not have to be performed in a laboratory and can be performed at a patient's bedside.

In accordance with the present invention, it has been determined that a wide variety of substances can be accurately and easily measured. These substances include any substance which is able to specifically interact with another substance. Such substances include immunogens, such as proteins, glycoproteins, nucleoproteins and large peptide hormones, such as insulin and growth hormone. These substances also include haptens such as drugs, vitamins, glycosides and polypeptides. Examples of other compounds which specifically interact with each other are lectins and sugars, enzymes and substrates, biotin and avidin, DNA and complementary DNA, RNA and complementary RNA, DNA and RNA and ligands and receptors for the ligands.

The principle of the solid phase diffusion assay is outlined in the following description using a radical diffusion competitive assay as an example. An adsorbent that is specific for a particular test substance is bound to an insoluble support such as nitrocellulose paper covered by a layer of water impermeable material such as a plastic sheet or tape, a hole being provided in the cover layer to expose the support. The diameter of the hole may, for example, be up to approximately 5 mm, e.g. between 1 and 5 mm.

Other suitable solid supports include thin layers chromatography plates which may be about 250 $\mu$m in thickness.

A solution of an unknown concentration of the test substance to which the adsorbent is specific is mixed with a known concentration of test substance labeled with colloidal gold or silver. A measured amount of the solution is applied to the exposed area of the insoluble support. The solution is allowed to diffuse in the insoluble support for several minutes. After diffusion is complete, the amount of diffusion is visualized. The diameter of the diffusion pattern on the solid support is proportional to the concentration of unlabeled test substance in the solution. The entire solid phase radial diffusion assay of the present invention takes only a few minutes to perform and the only measuring device required is a ruler.

Alternatively, if means are used to cause the sample to pass transversely through the support (as distinct from radially) for example by placing an absorbent layer beneath the support, all of the target substance will be immobilised in the exposed area of the support and the intensity of the colour is proportional to the quantity of immobilised target substance.

Numerous variations of this assay using the described basic principle may be performed. The test may be performed as a sandwich assay. In this case, only the soluble test sample is applied onto the solid phase with the adsorbent. The solid phase is then incubated in a solution containing the labeled adsorbent and the binding of the labeled adsorbent is visualized after a washing step.

A preincubation step to label the test substance directly can be performed. In this case, the test substance and the labeled adsorbents are incubated together and the mixture is applied to the solid with the adsorbents.

The solid phase diffusion assay of the present invention can be used to monitor a product of another assay. In this application, the solid phase diffusion assay of the present invention is used as a visualization step for assays measuring different substances.

In the solid phase diffusion assay of the present invention, the maximum amount of solution required to determine the concentration of a particular test substance in the solution is between approximately one to 50 $\mu$l. Thus, for example, if a blood antibiotic level is required, a finger prick would supply enough blood to perform the assay. Conventional methods of measuring blood antibiotic levels require that several cubic centimetres of blood be drawn from a venous puncture.

The invention is illustrated with reference to the following drawings in which:

FIGS. 1(a)-1(c) are schematic views of a solid phase diffusion assay of the present invention using only labeled test molecules

FIGS. 2(a)-2(c) are schematic views of a solid phase diffusion assay with labeled test molecules and unlabeled test molecules.

As used herein, the term "ligand" describes any compound for which a receptor naturally exists or can be prepared. The term "receptor" is used for any compound or composition capable of recognizing a particular spatial or polar organization of a molecule, i.e., epitopic site. Illustrative receptors include, but are not limited to, naturally occurring receptors, e.g., thyroxin binding globulin which will specifically bind thyroxin; Staphylococcal protein A which specifically binds immunoglobulins; antibodies; enzymes which specifically bind substrates; Fab fragments; lectins and the like.

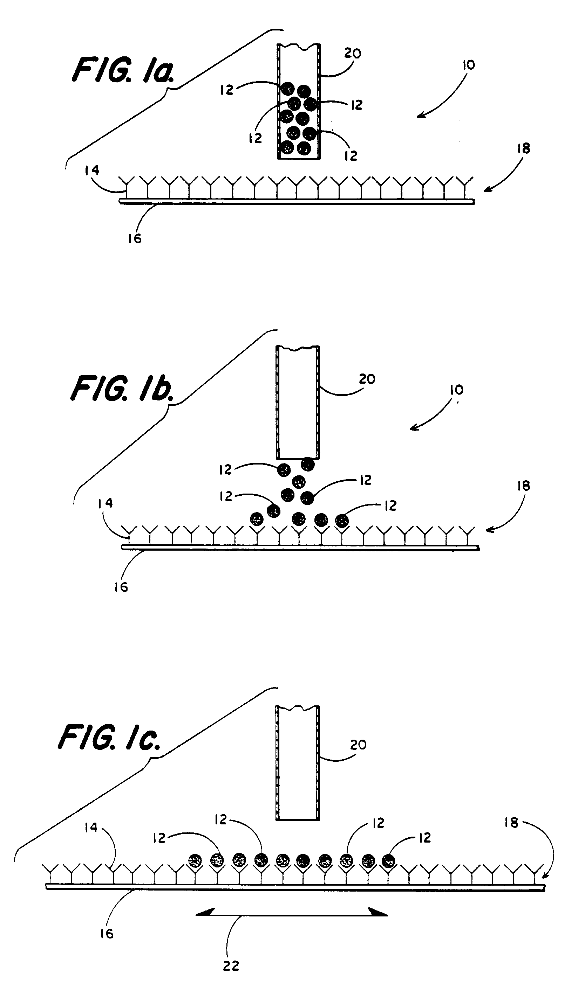

Referring now to the drawings in which like numbers indicate like elements throughout the several views, FIGS. 1(a)-1(c) show the binding of an unknown concentration of labeled test substance molecules in a radial diffusion assay. For simplicity, the cover layer is not shown. This figure shows the use of the solid phase diffusion assay of the present invention as a measurement step of a conventional assay. Shaded spheres represent labeled test substance molecules 12 in solution. These molecules may either be ligands, such as immunogens or haptens, or they may be receptors specific for the ligand. Examples of receptors are antibodies. Adsorbent molecules 14 can likewise be either antigens or receptors. The adsorbent molecules that are specific for the test substance molecules 12 are bound to a support 16 that is insoluble in the solvents that are used in the particular test. One example of an insoluble support is nitrocellulose paper (e.g. 0.45 microns in thickness). The insoluble support 16 is treated with the adsorbent molecules 14. For example, a typical adsorbent molecule that can be used in the present invention are antibodies that are specific for a particular antigen or hapten. The antibody molecule has an overall positive charge. The nitrocellulose paper has an overall negative charge. When the antibody molecules are applied in solution to the nitrocellulose paper, the positively-charged antibodies are ionically bound to the negatively-charged nitrate groups on the nitrocellulose paper. It is to be understood that other solid supports may be used and that the adsorbent molecules may be bound to the solid support either ionically or covalently. The adsorbent molecules 14 therefore provide specific binding sites on the support 16 to which the test substance molecules 12 can be bound. The insoluble support 16 treated with the adsorbent molecules 14 provides the solid phase 18 of the assay.

A known amount of test substance molecules 12 is applied to the solid phase 18 (via the hole in the cover sheet, not shown) by a capillary tube 20 or by other well known devices such as a micropipette or a microbiological loop. As shown in FIG. 1(b), when the test substance molecules 12 are applied to the solid phase 18, they diffuse radially outward from the point of application through the solid phase. As the test substance molecules 12 diffuse through the solid phase 18, the test substance molecules bind to the free adsorbent molecules 16 sites.

As shown in FIG. 1(c), when all the labeled test substance molecules 12 become bound to adsorbent molecules 14, diffusion of the test substance molecules through the solid phase 18 stops. The bound test substance molecules 12 provide a circular diffusion pattern on the solid phase. The circular diffusion pattern has a diameter such as at 22, which can be measured by well known techniques which will vary depending on the type of labeled substance used. The diameter of the diffusion pattern, will be proportional to the amount of labeled test substance in solution.

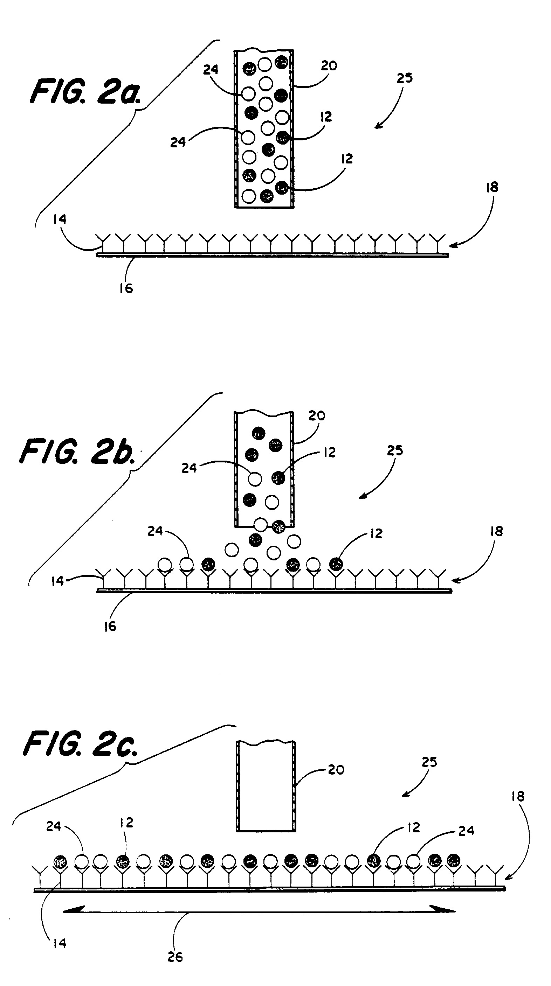

Referring to FIGS. 2(a)-2(c), there is shown a solid phase diffusion assay 25 of the present invention with an unknown amount of unlabeled test substance added to the solution of known labeled test substance. Again, the cover layer is not shown. This variant of the solid phase diffusion assay utilizes the principle of competition between the labeled test substance and the unlabeled test substance for binding sites on the solid phase. An unknown concentration of unlabeled test substance molecules 24, such as an antigen or a hapten, is mixed with a known concentration of labeled test substance molecules 14 to provide a test solution. The solid phase 18 is prepared as described above; however, the adsorbent molecules 14 are selected such that they will provide binding sites for both the labeled test substance molecules 14 and the unlabeled test substance molecules 24.

The test solution is applied to the solid phase 18 in the manner described above. The test solution diffuses radially outwardly from the point of application through the solid phase 18. Both the labeled test substance molecules 12 and the unlabeled test substance molecules 24 compete for binding with the free adsorbent molecules 14 binding sites. Diffusion of the test solution through the solid phase 18 continues until all of the labeled test substance molecules 12 and all of the unlabeled test substance molecules 24 are bound to be adsorbent molecules 14.

Because some of the binding sites are occupied by unlabeled test substance molecules 24, the labeled test substance molecules 12 will diffuse outwardly farther from the point of application than if no unlabeled test substance molecules were present. As a result, the diffusion pattern of the test solution has a diameter 26, FIG. 2(c) which is greater than the diameter 22, FIG. 1(c), of the diffusion pattern for the labeled test substance molecules 12 alone.

The distance that the labeled test substance travels is greater in FIG. 2c that in FIG. 1c because, in FIG. 2c, a percentage of the adsorbent binding sites are occupied by unlabeled test substance allowing the labeled test substance to diffuse further before encountering a free adsorbent binding site. Thus, the diameter of the diffusion pattern formed by the labeled test substance is proportional to the concentration of unlabeled test substance in the solution.

There are many variations of the solid phase diffusion assay of the present invention. For example, there are situations where the test substance either (a) cannot be labeled, or (b) where the affinity between the test substance and the receptors is too low to be used in the solid phase diffusion assay of the present invention or (c) where a very high sensitivity is desired or (d) where one may wish to use the same solid phase diffusion reagents to measure the concentration of different substances.

In the above situations, a preliminary step is required to measure the above test substances. In case (a) where the test substance cannot be labeled, the receptor for the test substance can be labeled and this receptor is then assayed in a final step of the solid phase diffusion assay of the present invention.

If the affinity between the test substance and the receptor is low, the test substance or the receptor can be conjugated with a high affinity ligand or receptor such as the biotin-(ligand)-avidin(receptor) system. The solid phase diffusion assay of the present invention may then be performed using the high affinity ligand and receptor.

Use of the same solid phase diffusion assay of the present invention for different test samples may be performed by linking biotin to the insoluble support and using labeled avidin as a ligand to measure different test substances. In this case, the labeled avidin is conjugated to antibodies against the different test samples. The test sample is then incubated with its complementary-labeled antibody and the antibody assayed in the biotin/avidin solid phase diffusion assay. Presence of the antigen diminishes the amount of labeled antibodies by crosslinking which will cause a diminution of the area of the diffusion pattern on the insoluble support.

The test solution in the solid phase diffusion assay of the present invention can be applied in several ways. The test solution can be applied to the treated support, exposed beneath the hole in the cover sheet, by a capillary tube, a micropipette or by a microbiological loop. The test solution can also be applied directly onto the plastic sheet or tape directly over the hole. The test solution will then diffuse through the hole and into the insoluble support thus increasing the amount of sample which may be applied to the exposed area of the support. Use of a cover sheet with a hole also facilitates application by a swab dipped in the sample.

The label conjugated to the test substance can also be indirectly linked to the test substance. For example, if the test substance is a hapten, it is possible to bind a protein to the hapten and then to conjugate the label to this protein. Alternatively, an antibody against the antigen can be labeled and used as the labeled antibody-antigen complex in the assay.

The different characteristics of the solid support strongly influence the performance of the assay. It is therefore possible to develop solid supports specially suitable for particular needs of the solid phase diffusion assay of the present invention. As a general rule, the thickness of the solid support is indirectly proportional to the amount of sample needed to cover a given surface and to the discriminatory capacity of the assay. The concentration of hydrophilic and hydrophobic components also influences the diffusion behaviour of the sample. The binding capacity of the solid support for the receptor is important to the sensitivity of the solid phase diffusion assay of the present invention. Methods for preparations of various solid supports are well known in one skilled in the art.

The solid phase insoluble supports useful in the present invention can be, for example, any support that has an overall negative charge so that an adsorbent molecule (either a receptor or a ligand) with an overall positive charge can bind non-covalently to the insoluble support. Examples of those types of supports are nitrocellulose paper, blotting membranes, diethylaminoethyl ion exchange paper and blot adsorbent filter paper. The solid phase insoluble supports can also be any support that has a functional group attached to the support to which an adsorbent molecule (either a receptor or a ligand) can be covalently attached. Example of these types of supports are aminobenzyloxymethyl (ABM) paper, 2-aminophenylthioether (APT) paper, cyanogen bromide activated paper (CBA) (See Methods in Enzymology, R. Win (ed.) 1979, Academic Press New York, 68:436-442 for a discussion of CBA paper), diazobenzyloxymethyl cellulose paper (DMB), diazophenylthioether cellulose paper (DPT) and nitrobenzyloxymethyl cellulose paper (NBM).

Methods that can be used to couple chemicals to the solid phase support depend, in part, on the chemical composition of the support and the chemical composition of the chemical to be coupled to the support. Chemicals can be coupled to a support by use of cyanogen bromide coupling, silation, diazo coupling, carbodiimide coupling, glutaraldehyde coupling and the use of heterobifunctional reagents. In many cases, due to stereochemical inhibition, spacer groups are required to couple one chemical to another chemical. Common spacer groups include, but are not limited to, diamino alkyl or aryl groups, aryl carboxyclicic acid or gamma amino alkyl groups, thiol, hydroxyl and mercurated bases.

The exclusion limit dictated by the pore size of the insoluble support will determine the size of the particle that can be diffuse in the solid phase. The pore size of the solid phase can be utilized to eliminate a separation step in an assay. For example, when heparinized blood is analyzed, the cellular components of the blood must usually be separated from the fluid or plasma portion of the blood before any analysis can be performed. This is usually done by centrifugation. With a solid phase radial diffusion assay of the present invention, this centrifugation step can be eliminated because the pore size of the insoluble support can be selected to block the diffusion of the cellular components of the blood.

In a further variation of this embodiment of the solid phase diffusion assay of the present invention, the test solution may be applied to the insoluble support through a filter. The test solution is applied to the top of the filter and the test solution diffuses through the filter and into the insoluble support. Examples of typical filters include, but are not limited to, blotting paper and diethylaminoethyl ion exchange paper. An example of using this procedure is in separating blood cells from plasma where the insoluble support would lyse the erythrocytes in whole, heparinized blood. The released hemoglobin from the lysed cells would cause a high background color in the insoluble support and make the visualization of the diffusion pattern difficult.

The unlabelled test substances that can be assayed by the solid phase diffusion assay of the present invention include, but are not limited to, the class of substances known as antigens. Antigens can be broken down into two groups: immunogens and haptens.

Immunogens are compounds which, when introduced into a chordate, will result in the formation of antibodies. Representative of the immunogens are proteins, glycoproteins and nucleoproteins, such as peptide hormones, serum proteins, complement proteins, coagulation factors, and viral or bacterial products. Certain body compounds with ubiquitous presence in all animal species cannot be used to produce antibodies because these compounds are not recognized as foreign by the immunized animal. These compounds can be rendered "foreign" by chemical derivation. The test substance in an assay has to undergo the same derivation procedure if antibodies against an altered compound are used.

Table III is a partial list of some of the types of immunogens that can be quantitated by the solid phase diffusion assay of the present invention.

Table III

| | |
|---|---|
| nucleoproteins | peptide hormones |
| serum proteins | complement proteins |
| coagulation factors | microbiocidal products |
| viral products | bacterial products |
| fungal products | specific Immunogens |
| albumin | angiotensin |
| bradykinin | calcitonin |
| carcinoembryonic antigen | chloriomamotropin |
| chorogonadotropin | corticotropin |
| erythropoietin | Factor VIII |
| fibrinogin | alpha-2-H globulin |
| follitropin | Gastrin |
| gastrin sulfate | glucagon |
| gonadotropin | haptoglobin |
| Hepatitis B surface antigen | immunoglobulins (A,D,E,G,M) |
| insulin | lipotropin |
| kallidin | lipotropin |
| melanotropin | oxytocin |
| pancreozymin | placental lactogen |
| pyrathryin | proangiotensin |
| prolactin | somatotropin |
| relxin | secretin |
| somatomadin | somatostatin |
| thryrotropin | vasotocin |
| thymopoietin | vasopressin |
| alpha-1-fetoprotein | alpha-2-H globulin |

Haptens are compounds which, when bound to an immunogenic carrier and introduced into a chordate, will elicit formation of antibodies specific for the hapten. Representative of the haptens are steroids such as estrogens and cortisones, low molecular weight peptides, other low molecular weight biological compounds, drugs such as antibiotics and chemotherapeutic compounds, industrial pollutants, flavoring agents, food additives, and food contaminants, and/or their metabolites or derivatives.

The above classes are obviously incomplete in that the solid phase diffusion assay of the present invention can be used to assay for any molecule to which an antibody can be formed. In addition, the solid phase diffusion assay of the present invention can be used to identify and quantitate an antibody molecule.

An antibody that can be used in the solid phase diffusion assay of the present invention can be produced by introducing the antigen to be assayed, if it is an immunogen, into a living chordate. The antibodies, which are produced in response to the introduction of the immunogen, are proteins that coat the immunogen and detoxify it, precipitate it from solution, or simply bind to it. The antibody protein forms a receptor which is geometrically arranged so that the immunogen fits the spatial arrangement of the protein. In the case of a hapten, an extra step is involved in preparing the antibody. The hapten must be conjugated to an immunogenic carrier prior to introduction into a living vertebrate. The method of preparing the antibodies from haptens is well known to those skilled in the art.

Another source of antibodies that can be used in the solid phase diffusion assay of the present invention is mono-clonal antibodies. The technique for producing monoclonal antibodies involves the fusing of spleen lymphocytes with malignant cells of bone marrow primary tumors. The method creates a hybrid cell line, arising from a single fused cell hybrid, or clone, which possesses characteristics of both the lymphocytes and myeloma cell lines. Like the lymphocytes (taken from animals primed with the particular antigen), the fused hybrids, called hybridomas, secrete a single type of immunoglobulin specific to the antigen; moreover, like the myeloma cell lines, the hybrid cell line is immortal. The combination of these two features has had a major impact in fields of research and medicine in which conventional antisera are used. Whereas antisera derived from vaccinated animals are variable mixtures of antibodies which never can be reproduced identically, monoclonal antibodies are highly specific immunoglobulins of a single type. The single type of immunoglobulin secreted by a hybridoma is specific to one and only one antigenic determinant on the antigen, a complex molecule having a multiplicity of antigenic determinants. (See C.

Milstein, Scientific American, 243(4):66-74, 1980) Any system where there is a specific interaction among substances can be used in the solid phase diffusion assay of the present invention. Examples of systems other than the antibody/antigen system which are useful in the present invention include lectins/sugar systems, enzyme/substrate, hybridization of DNA and RNA molecules, the biotin/avidin system and Staphylococcal protein A/immunoglobulin system.

As a matter of convenience, the reagents for the solid phase diffusion assay can be provided as kits, where the reagents are in predetermined ratios, so as to substantially optimize the sensitivity of the assay in the range of interest. After reconstitution of dry reagents, in predetermined volumes, the concentration of the reagents will be at appropriate levels.

According to a further feature of the invention we provide a kit for the qualitative or quantitative determination of analyte in a test sample said kit comprising:

(a) an insoluble porous sheet support to which is bound a first substance selected from the group consisting of ligands and receptors, specific for said analyte said support being covered by a layer of water-impermeable material having a hole therein and/or having adjacently beneath a sheet of hydrophilic material serving to enhance transverse diffusion of liquid through said support, and

(b) a solution of a second substance, said second substance being selected from the group consisting of ligands and receptors binding to said first substance or said analyte, said second solution comprising a known concentration of said second substance conjugated to a label comprising colloidal gold or colloidal silver.

The present invention is illustrated further by the following examples which are not to be construed as limiting the invention to the specific procedures described in them.

Example 1

A mixture of three monoclonal antibodies against the alpha subunit of human chorionic gonadotropin (HCG) is labeled with colloidal gold using the method described in J. DeMay, Colloidal Gold Probes in Immunocytochemistry, Immunocytochemistry: Applications in Pathology and Biology, Ed.: J. Polak, S. Van Noorden, J. Wright & Sons Ltd., London, pgs. 82-112, 1983. A nitrocellulose membrane (BioRad, Rockville Centre, NY, No. 162-0115, 0.45 $\mu$m) is saturated with polyclonal antibodies against HCG which have been produced in a rabbit and which have been affinity purified in a Staphylococcal protein A column as known to one skilled in the art. The membrane is then covered with a cover having an approximately 2 mm$^2$ aperture therein. A swab containing lyophilized gold-labeled anti-HCG antibodies is wetted with the sample urine which may contain HCG. The swab is then immediately brought into contact with the membrane cover. The urine diffuses from the swab through the opening in the membrane cover and into the nitrocellulose membrane. The swab is held in place for about 30 second and then removed. Concentrations of HCG greater than 50 mIU/mL, which generally indicate a pregnancy, can be diagnosed by the presence of a red spot. Concentrations of HCG below 50 mIU/mL will not produce a visible spot.

Example 2

The procedure of Example 1 was repeated, using a negative pressure (e.g. vacuum) applied to the underside of the nitrocellulose paper. The negative pressure was applied to enhance diffusion of the test solution (e.g. urine) through the nitrocellulose paper. The nitrocellulose paper was covered on both sides with adhesive tape, i.e., it was provided with a cover which limited the area of contact between the sample and the membrane. The tape has a 2 mm$^2$ aperture on corresponding locations on both sides of the membrane which allowed for the concentration of the analyte-labeled antibody complex on a very small surface. The presence of the analyte could be seen as a red spot at the place of application.

Example 3

The procedure of Example 2 was repeated, using a positive pressure to enhance diffusion instead of a negative pressure. The positive pressure was produced using a 1 mL syringe containing 0.5 mL of the analyte/gold-labeled antibody mixture. The covered membrane contained corresponding 2 mm$^2$ openings in the covering on each side of the membrane. The membrane and its covering on both sides were housed in a standard sterile filter housing whose membrane had been replaced by the above-described membrane and covering. The syringe was connected to the filter housing and the analyte/gold-labeled antibody mixture was forced through the area of the membrane exposed by the corresponding openings. The presence of the analyte could be seen as a red spot at the place of application.

Example 4

The procedure in Example 2 was repeated using a hydrophilic material to enhance diffusion instead of a negative pressure. The hydrophilic material was located adjacent to one side of the membrane covering. The analyte/gold-labeled antibody mixture was pipetted onto the area of the opening on the side opposite the hydrophilic membrane and allowed to diffuse successively through the opening, the paper, and into the hydrophilic membrane. The presence of the analyte was visualized by a red spot.

It should be understood that preceding Examples 2, 3 and 4 can be carried out using a membrane which is covered only on the side where the sample is applied. However, if only one side is covered, there will likely be greater lateral diffusion of the analyte/gold-labeled antibody mixture.

The following Referential Examples illustrate the application of immunodiffusion assays to a number of analytes using labels other than colloidal gold or silver and/or omitting the use of a layer over the porous support.

Referential Example I

The following referential example demonstrates a solid phase diffusion assay as used to detect small quantities of inactivated horse radish peroxidase. This is an example of a competitive assay based on an antibody/ antigen interaction where the competitive compound by itself is used as the label. Antiperoxidase antibodies are bound to the solid-phase which, in this example, is nitrocellulose paper. Inactivated horseradish peroxidase is the antigen and active peroxidase corresponds to the labeled antigen in this assay.

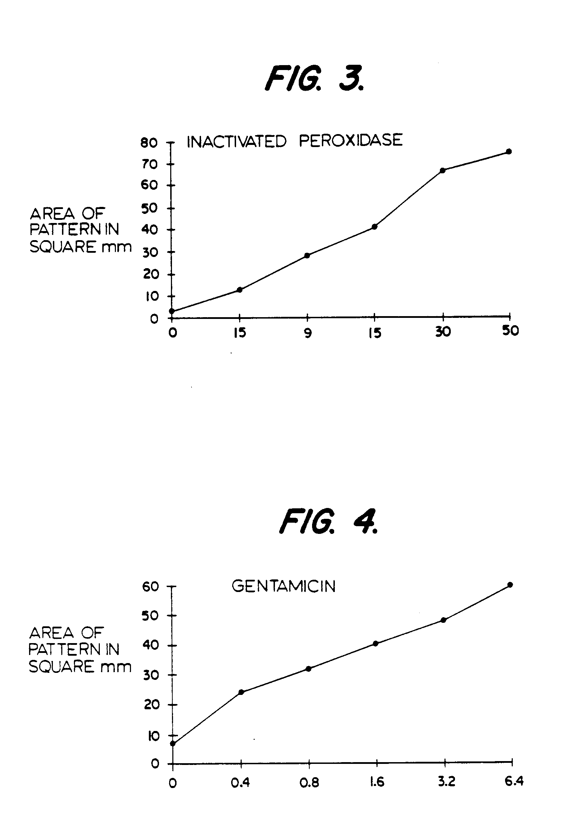

A standard curve is prepared by preparing a concentrated solution of inactivated horseradish peroxidase. The concentration of active peroxidase (the label in this case) was determined as follows. Several dilutions of a solution with 1 mg/ml of peroxidase were mixed with ten percent rabbit serum and phosphate buffered saline (no test solution) and were applied to the treated nitrocellulose. The highest dilution that still provides a measurable diffusion patter was used as the label in this example. The solution of inactivated horseradish peroxidase (fifty $\mu$g/ml) is serially diluted in phosphate buffered saline containing ten percent rabbit serum. 2 1/2 $\mu$l of each of the solutions of inactivated horseradish peroxidase is mixed with 2 1/2 $\mu$l of a solution containing 0.3 $\mu$g of the active peroxidase (the labeled antigen in this example). The 5 $\mu$l of solution is then carefully applied by diffusion from a capillary tube to the nitrocellulose papers containing the bound anti-peroxidase antibodies. The solution diffuses from the capillary tube into the nitrocellulose paper and forms a circular diffusion pattern. The nitrocellulose papers are then immersed in a solution of horseradish peroxidase substrates (4-chloro-1-naphthol and hydrogen peroxide) and incubated until a blue circle developed. As shown in FIG. 3, the area of the circular diffusion pattern is proportional to the amount of unlabeled antigen in the solution. It has been found that only a single standard curve has to be run for a given set of antibody and labeled antigen reagents. The detailed procedure of the solid phase diffusion assay is as follows:

The nitrocellulose paper (Bio-Rad, Rockville Centre, NY, No. 162-0115, 0.45 $\mu$m) is cut into pieces with a dimension of approximately 6.45 $cm^2$ (1 square inch). These pieces are then washed for 10 minutes in phosphate buffered saline (pH 7.2). The washed papers are then incubated at 4°C for 12 hours in a solution containing 10 mg/ml rabbit anti-peroxidase immunoglobulin G (Batch C1, affinity chromatography purified). After the 12 hour incubation, the papers are again washed for 10 minutes in phosphate buffered saline. The papers are then incubated for 2 hours in a solution of 5% serum albumin. This step is performed to saturate all non-specific binding sites. The papers are again washed in phosphate buffered saline. After a short wash in distilled water the membrane pieces are air dried and stored at room temperature in a humid chamber.

The antigen that is used in this example was inactivated horseradish peroxidase. This antigen is prepared by dissolving 1.5 mg horseradish peroxidase (Type VI, Sigma Chemical Company, St. Louis, MO, No. P-8375, Lot 43F-9598) in phosphate buffered saline. The enzyme is inactivated by adding hydrogen peroxide to a final concentration of 1.0% and then dialyzed against phosphate buffered saline overnight.

2.5 $\mu$l of the sample containing the unknown horseradish peroxidase antigen is mixed with 2.5 $\mu$l of a solution containing 0.3 $\mu$g of activated peroxidase. The 5 $\mu$l solution is carefully applied to an antibody-treated nitrocellulose paper by diffusion from a capillary pipet. The substrate solution is made up as follows: 15 mg of 4-chloro-1-naphthol (Bio-Rad, Rockville Centre, NY, No. 170-6534) is dissolved in 5 ml of methanol. To this solution is added 25 ml of distilled water and 15 $\mu$l of methanol. To this solution is added 25 ml of distilled water and 15 $\mu$l of 30% hydrogen peroxide. A blue circular pattern develops after several minutes.

To determine the concentration of antigen in the test solution, the area of the circular pattern is measured. By using the standard curve, an accurate value for the concentration of antigen in the solution can be determined.

FIG. 3 shows the relationship between the area of the diffusion pattern and the concentration of unlabeled, inactivated peroxidase in the test samples.

Referential Example II

This referential example demonstrates a solid phase diffusion assay as used to detect low concentrations of the antibiotic gentamicin in solution. This is an example of a competitive assay based on an antigen/antibody interaction where the test substance is a hapten and the labeled compound comprises a hapten bound to a carrier to which the label is bound.

The nitrocellulose paper (Bio-Rad, Rockville Centre, NY, No. 162-0115, 0.45 $\mu$m) is cut into pieces with a dimension of approximately 6.45 cm$^2$ (1 square inch). These pieces are then washed for 10 minutes in phosphate buffered saline (pH 7.2). The washed papers are then incubated at 4°C for 12 hours in whole goat serum containing goat anti-gentamicin antibodies diluted 1:3 in phosphate buffered saline. No saturation step is required in the Referential Example due to the high protein concentration of the diluted goat serum. The papers are then washed in phosphate buffered saline. After a short wash in distilled water the membrane pieces are air dried.

The gentamicin is chemically linked to bovine orosomucoid using the carbodiimide coupling procedure which is well known to those skilled in the art. After the gentamicin is linked to the orosomucoid, horseradish peroxidase is then linked to the orosomucoid protein using the glutaraldehyde method (see S. Avrameas, Immunochemistry, Vol. 1 6:43, 1969). This procedure produces a complex made up of orosomucoid-gentamicin-horseradish peroxidase complex.

The orosomucoid-gentamicin-horseradish peroxidase complex is purified using the procedure of affinity chromatography. One gram of cyanogen bromide activated Sepharose® 4B (Pharmacia Fine Chemicals, Upsula, Sweden) is washed in 1 mM HCl. 10 mg/ml of the gentamicin-orosomucoid-horseradish peroxidase is then covalently coupled to the Sepharose® 4B using the manufacturers standard protocol. The resulting gel is then poured into a small chromatography column (Economo column, Bio-Rad). The goat anti-gentamicin antibodies are then adsorbed onto the column by passing 5 ml of the goat anti-gentamicin serum diluted 1 to 10 in phosphate buffered saline through the column. The antibody is next covalently linked to the solid phase by incubation with a 0.02 M glutaraldehyde solution during 2 hours at room temperature. Free binding sites of the glutaraldehyde are saturated with glycine buffer and the column is then extensively washed with phosphate buffered saline.

The affinity column is then used for purification of the gentamicin-orosomucoid-peroxidase complex. O.1 M HCl and 0.2 M glycine at a pH of 2.5 is used for elution of the labeled complex. The pH of the eluate is immediately corrected by adding solid Tris (Tris(hydroxymethyl)-aminomethane, Sigma Chemical Company, St. Louis). The resulting solution of purified gentamicin-orosomucoid- peroxidase complex is then dialyzed against phosphate buffered saline before storage.

The solutions for determining the standard curve are prepared in phosphate buffered saline, 10% normal rabbit serum, containing 6 different gentamicin dilutions. The concentrations of gentamicin in the standard curve range between 0.4 $\mu$g/ml to 12.4 $\mu$g/ml. The protein concentration of the labeled gentamicin-orosomucoid-peroxidase complex is approximately 0.3 mg as determined by the absorbence of the solution at 280nm. 5 $\mu$l of each dilution is applied with a capillary tube onto the nitrocellulose paper. After the test fluid diffuses into the nitrocellulose paper is then submerged in a substrate solution. The substrate solution is made up as follows: 15 $\mu$g of 4-chloro-1-naphthol (Bio-Rad, Rockville Centre, NY, No. 170-6534) is dissolved in 5 ml of methanol. To this solution was added 25 ml of distilled water and 15 $\mu$l of 30% hydrogen peroxide. A blue circular pattern develops after several minutes.

FIG. 4 shows the relationship between the area of the diffusion pattern and the concentration of unlabeled, gentamicin in the test samples.

Referential Example III

The following referential example demonstrates a solid phase diffusion assay as used to detect low concentrations of the drug Theophylline. This is another example of an antigen-antibody interaction where the test substance is a low molecular weight hapten and the labeled compound comprises a hapten bound to horse radish peroxidase. This test also uses a monoclonal antibody as opposed to a heterogeneous antibody.

The nitrocellulose paper (Bio-Rad, Rockville Centre, NY, No. 162-0115, 0.45 $\mu$m) is prepared as

described in Referential Examples I and II. The washed papers are then incubated in phosphate buffered saline containing a mixture of 10 $\mu$g/ml of mouse monoclonal antibody against Theophylline and 2 mg of bovine serum albumin (Sigma Chemical Company, St. Louis) overnight at 4°C. The papers are then washed in phosphate buffered saline, air dried and stored in a humid chamber.

The Theophylline is conjugated to horse radish peroxidase. (See theophylline radioimmunoassay: Synthesis of Antigen and Characterization of Antiserum, C.E. Coole, et.a., Research Communications in Chemical Pathology and Pharmacology, Vol 13, No. 3, 1976.) The Theophylline/horse radish peroxidase conjugate is purified using affinity chromatography by the same procedure as described in Referential Example II using the monoclonal anti-Theophylline antibody.

A standard curve is prepared containing six different Theophylline dilutions in phosphate buffered saline and 10% rabbit serum. The concentrations of Theophylline range between 1.6 and 25.6 $\mu$g/ml. A mixture containing 2 1/2 $\mu$l of a 1 to 2 dilution of the labeled antigen in 10% rabbit serum and 2 1/2 $\mu$l of each dilution is applied with a capillary tube onto the nitrocellulose paper. After diffusion of the fluid into the nitrocellulose paper, the paper is submerged into the above described substrate solution and the color reaction allowed to develop. The diameters of the diffusion patterns are then measured and the area of the diffusion pattern is calculated.

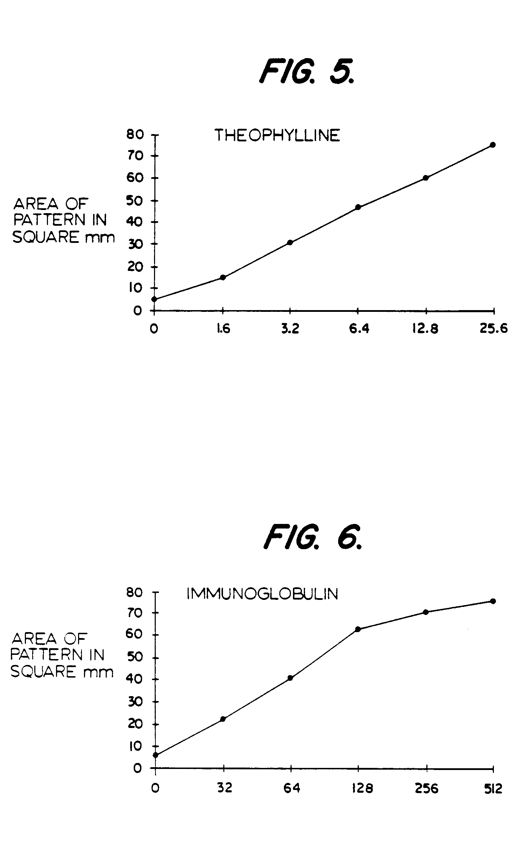

FIG. 5 shows the relationship between the area of the diffusion pattern and the concentration of unlabeled Theophylline in the test samples.

## Referential Example IV

The following referential example demonstrates a solid phase diffusion assay as used to detect low concentrations of human immunoglobulins reacting with Staphylococcal Protein A. This is an example of a "sandwich assay" based on a ligand (immunoglobulin) and receptor (Protein A) interaction. Peroxidase labeled rabbit antibodies directed against the human immunoglobulins are used as free antibodies.

The nitrocellulose paper (Bio-Rad, Rockville Centre, NY, No. 162-0115, 0.45 $\mu$m) is cut into pieces with a dimension of approximately 6.45 cm$^2$ (1 square inch). These pieces are then washed for 10 minutes in phosphate buffered saline (pH 7.2). The washed papers are then incubated at 4°C for 12 hours in a phosphate buffered saline solution containing 0.01 mg/ml Staphylococcal Protein A (Pharmacia Fine Chemicals, Uppsala, Sweden) and 1 mg/ml bovine serum albumin (Sigma Chemical Company, St. Louis, MO). After the 12 hour incubation, the papers are again washed for 10 minutes in phosphate buffered saline. The papers are then incubated for 2 hours in a 5% solution of bovine serum albumin. This incubation is performed to saturate non-specific binding sites. The glycine incubation is performed to saturate all non-specific binding sites. The papers are again washed in phosphate buffered saline. After a short wash in distilled water the membrane pieces are air dried.

A standard curve is prepared in phosphate buffered saline containing 5% bovine serum albumin using 6 different human immunoglobulin G dilutions. The concentrations of immunoglobulin G (Boehringer, Mannheim, Germany) in the standard curve range between 32 $\mu$g/ml to 1 mg/ml. A solution containing 10 $\mu$l of each dilution was applied with a capillary tube onto the nitrocellulose paper. After the test fluid diffuses into the nitrocellulose paper, the papers are then washed for 3 minutes in a phosphate buffered saline solution containing 0.5% Tween® 20 (polyoxyethylenesorbitan monolaurate, Sigma Chemical Company, St. Louis, Mo). Thereafter, peroxidase labeled antibodies specific for human IgG heavy and light changes (Dako Accurate Chemicals) are applied as a second layer of the sandwich. These labeled antibodies are diluted 1:1000 in phosphate buffered saline with 1% bovine serum albumin.

After a second washing step using phosphate buffered saline and 0.5% Tween® 20, the papers are then submerged in a substrate solution. The substrate solution is made up as follows: 15 $\mu$g of 4-chloro-1-naphthol (Bio-Rad, Rockville Centre, NY, No. 170-6534) was dissolved in 5 ml of methanol. To this solution is added 25 ml of distilled water and 15 $\mu$l of 30% hydrogen peroxide. A blue circular pattern develops after several minutes.

As shown in FIG. 6, the area of the circular diffusion pattern is proportional to the amount of free immunoglobulins in the test solution. It has been found that only a single standard curve has to be run for a given batch of prepared nitrocellulose test substances and labeled antibody.

## Referential Example V

Solid phase supports available for thin layer chromatography can be utilized in solid phase diffusion assay. The commercially available thin layer chromatography supports are very thin, usually about 250 $\mu$g thick and may easily be adapted to the solid phase diffusion assay of the present invention.

Anti-gentamicin antibodies are covalently bound to the solid support in the following manner. An avicel F chromatography plate, is incubated overnight at 4°C with the goat anti-gentamicin serum diluted 1:4 with phosphate buffered saline and 50 $\mu$l of glutaraldehyde per 100 ml of solution. The plate is then extensively washed with phosphate buffered saline with 1% bovine serum albumin (Sigma Chemical Company, St. Louis, MO) and finally with phosphate buffered saline alone. The plate is then air dried.

The assay is performed by adding 20 $\mu$l in four different dilutions of the gentamicin-horse radish peroxidase-orosomucoid conjugate described in Referential Example II to a single point on the thin layer chromatography plate. After the solution diffuses, the enzyme substrate is added as described in the previous examples.

Referential Example VI

The following referential example shows the application of the solid phase diffusion assay of the present invention as the final step of a multistep assay. This assay is designed to measure the concentration of human Immunoglobulin G. One $\mu$g of affinity-purified peroxidase-labeled antibody specific for human immunoglobulin G (Dako Accurate Chemicals, Westbury, New York) is incubated for fifteen minutes at room temperature in 100 $\mu$l of phosphate buffered saline, 10% rabbit serum, together with 10 $\mu$l of a 1 to 1000 dilution of the test serum (diluted in phosphate buffered saline. Five $\mu$l of the test substance is then applied to the nitrocellulose coated with rabbit anti-peroxidase antibody. This nitrocellulose was prepared as described in Referential Example I with the following difference. The rabbit immunoglobulin was diluted with normal rabbit serum so that 4 $\mu$l containing 1 $\mu$g of the above peroxidase-labeled antibody diffuses close to the edge of the diffusing solution (approximately 8mm). Thus, in this variation of the solid phase diffusion assay of the present invention, the diffusion pattern of the reagents added with no test solution will have the largest area. If the test solution contained any human immunoglobulin G, the immunoglobulin G molecules will react with the peroxidase-labeled antibodies in the solution. Since a single immunoglobulin G specific antibody will react with more than one immunoglobulin G molecule, there is extensive crosslinking between immunoglobulin molecules as the binding reaction proceeds. Thus, large complexes will reduce the number of free peroxidase labeled antibodies in solution and will also increase the size of diffusing complexes. Thus, the size of the diffusion pattern is markedly diminished as the concentration of human immunoglobulin G molecules in the test solution is increased. A standard curve is prepared with gradually increasing concentration of human immunoglobulin G in the test solution.

Referential Example VII

The following example shows the application of a solid phase diffusion assay as used as the final step of an assay to qualitatively and quantitatively analyze the end product. This approach may be chosen for a substance where no receptors of high affinity can be found, where only very special labels can be used or where a very high sensitivity may be necessary.

For example, it has proved difficult to produce an antibody with high enough affinity for this application against Clostridium perfringens toxin. Thus it would be difficult to perform the solid phase diffusion assay as described in Referential Examples I-IV since the antibody to the toxin is of low affinity. This variation of the solid phase diffusion assay will allow one to perform a solid phase diffusion assay using the low affinity antibodies.

Rabbit antibodies against the toxin are labeled with peroxidase and then affinity-purified as is well known to one skilled in the art. The nitrocellulose paper is treated with antibodies specific for horseradish peroxidase. A constant amount of the peroxidase-labeled antibody is then incubated with the unknown amount of perfringens toxin. The mixture of labeled perfringens toxin antibody and unknown perfringens toxin is then applied to a single point on the insoluble support and allowed to diffuse. Thus, as in Referential Example VI, in this variation of the solid phase diffusion assay, the diffusion pattern of the reagents added with no test solution will have the largest area.

Since a single toxin-specific antibody will react with more than one toxin molecule, there is extensive crosslinking between toxin molecules as the antibody-toxin molecules and peroxidase-labeled toxin specific antibodies. This cross-linking will therefore reduce the number of free toxin antibodies in solution and will also increase the size of diffusing complexes. Thus, when the toxin-antibody-peroxidase complexes are applied to the peroxidase-specific antibody-treated nitrocellulose paper, the size of the diffusing pattern is markedly diminished as the concentration of toxin molecules in the test solution is increased. A standard curve is prepared with gradually increasing concentration of Clostridium perfringens toxin in the test solution.

Referential Example VIII

In this referential example, the reagents and the insoluble support are prepared as in Example IV. A thin plastic sheet is prepared with a hole punched in the center of the sheet. The diameter of the hole is 2 mm. The plastic sheet is then placed on the nitrocellulose paper so that the hole is positioned approximately in the center of the nitrocellulose paper. 10 $\mu$l of test solution is placed over the hole in the plastic. The test solution diffuses through the hole in the plastic and into the nitrocellulose paper. After the diffusion is complete, the substrate is added and the diffusion pattern is measured as described in the previous referential examples.

Referential Example IX

Horse radish peroxidase is labeled with the colloidal gold according to a procedure described in J. DeMay, Colloidal Gold Probes in Immunocytochemistry, Immunocytochemistry; Applications in Pathology and Biology, Ed.: J. Polak, S. Van Noorden, J. Wright & Sons Ltd., London, pgs. 82-112, 1983.

Nitrocellulose paper is prepared as described in Referential Example I. A standard curve measuring non-labeled peroxidase is then prepared as in Referential Example I. 2 and 1/2 $\mu$l of non-labeled peroxidase in concentrations of 2,4,6,8 and 10 $\mu$g/ml are added to the same quantity of 1 $\mu$g/ml of colloidal gold labeled peroxidase and a standard curve is established as described in Referential Example 1. The circle that indicates the diffusion of the sample is immediately visible. No subsequent incubations are required to visualize the diffusion pattern.

Referential Example X

This example illustrates the use of a solid phase diffusion assay for qualitative and quantitative detection of Deoxyribonucleic acid (DNA) or Ribonucleic acid (RNA). This example allows for rapid quantitation of Chlamydia trachomatis DNA in a sample.

A. Preparation of the nitrocellulose filter: A 10 microgram/mL solution of a 0.1 kilobase DNA probe for Chlamydia trachomatis in 0.1 M NaOH, 1 M NaCl, 150 mM Na Citrate is heat denatured by boiling for 3 minutes and applied to 2 cm$^2$ of nitrocellulose paper (Bio-Rad as described). The paper is then floated for 10 seconds in a solution of 1 M Phosphate Buffer pH 7 to neutralize the NaOH. The filter is then baked in a vacuum oven for 10 minutes at 80°C. Unreacted sites are blocked by incubation overnight in a DNA blocking buffer as described in "Molecular Cloning, a laboratory, Manual, eds. T. Maniatis, E.F. Fritsch and J. Sambroock, Cold Spring Harbor Laboratory, 1982, page 326.

B. Detection of sample nucleic acid: Ten microliters of the sample nucleic acid is mixed with 10 microliters of 0.2 $\mu$g/mL solution of a second Chlamydia trachomatis DNA probe in DNA blocking buffer. This second DNA probe is labeled with biotin as known to one skilled in the art. The biotin-labeled DNA probe sequences are not complementary to those of the solid phase probe. The mixture of sample nucleic acid and labeled probe is then heat denatured by boiling for 3 minutes. Five microliters of the mixture are applied with a capillary micropipette to the nitrocellulose and allowed to diffuse out radially. The biotin-labeled DNA probe is then visualized by the application on exactly the same spot with a capillary micropipette of five microliters of a 0.001 mg/mL solution of colloidal avidin-gold 15 nm particles (EY laboratories, San Mateo CA, catalogue number GA-01) in phosphate buffered saline pH 7.4. The formation of a red spot indicates the presence of Chlamydia trachomatis DNA in the sample.

**Claims**

1. A method for the quantitative or qualitative determination of an analyte in a liquid test sample comprising the steps of:

   (a) binding a first substance to an insoluble porous sheet support said first substance being selected from the group consisting of ligands and receptors binding said analyte, said support being covered by a layer of water-impermeable material having a hole therein and/or having adjacently beneath a sheet of hydrophilic material serving to enhance transverse diffusion of liquid through said support;

   (b) applying said test sample to said insoluble support, through said hole, where said cover layer is present;

   (c) permitting said test sample to diffuse through said insoluble support carrying said first substance;

   (d) applying a labeled second substance to said insoluble support before, together with, or after application of said analyte, said second substance being selected from the group of ligands and

16

EP 0 207 152 B1

receptors capable of being immobilized by binding to either said first substance or said analyte and being labeled with colloidal gold or colloidal silver; and
(e) assessing the presence and/or quantity of said immobilized labeled second substance.

2. A method according to claim 1, wherein for qualitative determination said label is present in amount sufficient to provide a clear signal which is visible to the naked eye when said second substance is present in an amount above a predetermined detection limit, and which is not visible to the naked eye when said second substance is present in an amount below said predetermined detection limit.

3. A method according to claim 1 in which said hydrophilic sheet is absent and, for quantitative determination the analyte in the test sample is allowed to diffuse laterally through said insoluble support to provide a radial diffusion pattern and the diameter of said diffusion pattern is measured.

4. A method according to claim 1 or claim 2 wherein said cover layer of water impermeable material is present and diffusion of the test sample transversely through said insoluble support is enhanced by employing a negative pressure or positive pressure or by providing said hydrophilic material positioned on the side of said insoluble support opposite to said hole.

5. A method according to any of claims 1 to 4 in which the test sample contains an unknown concentration of the analyte and a known concentration of said labeled second substance, said second substance being capable of binding to said immobilised first substance, whereby a competitive binding assay is effected.

6. A method according to any of claims 1 to 4 in which said labeled second substance is capable of binding to said analyte and is applied to said support after said analyte.

7. A method according to any of claims 1 to 6, wherein said second substance is labeled by means of another labeled compound, said compound belonging to the group of ligands and receptors binding to said second substance.

8. A method according to any of claims 1 to 6 in which the diameter of said hole is up to approximately 5mm.

9. A method according to any of claims 1 to 7 wherein said first substance is directly or indirectly, covalently or non-covalently bound to said insoluble support.

10. A method according to any of claims 1 to 8 wherein said ligands and receptors are chosen from the groups of specifically interacting antigens and antibodies, sugars and lectins, nucleic acids, enzymes and substrates, enzymes and inhibitors, biotin and avidin or complexes thereof, or immunoglobulin and Staphylococcal Protein A or complexes thereof.

11. A method according to any of claims 1 to 9 wherein said insoluble support is a polymeric material carrying functional groups capable of being bound to proteins, carbohydrates or nucleic acids.

12. A kit for the qualitative or quantitative determination of analyte in a test sample said kit comprising:
(a) an insoluble porous sheet support to which is bound a first substance selected from the group consisting of ligands and receptors, specific for said analyte said support being covered by a layer of water-impermeable material having a hole therein and/or having adjacently beneath a sheet of hydrophilic material serving to enhance transverse diffusion of liquid through said support, and
(b) a solution of a second substance, said second substance being selected from the group consisting of ligands and receptors binding to said first substance or said analyte, said second solution comprising a known concentration of said second substance conjugated to a label comprising colloidal gold or colloidal silver.

13. A kit according to claim 11 wherein where said cover layer is present the diameter of said hole is up to 5mm.

14. A kit according to claim 11 or claim 12 wherein said first substance is directly or indirectly, covalently

17

or non-covalently bound to said insoluble support.

**15.** A kit according to any of claims 11 to 13 wherein said ligands and receptors are chosen from the groups of specifically interacting antigens and antibodies, sugars and lectins, nucleic acids, enzymes and substrates, enzymes and inhibitors, biotin and avidin or complexes thereof, or immunoglobulin and Staphylococcal Protein A or complexes thereof.

**Patentansprüche**

**1.** Verfahren zur quantitativen oder qualitativen Bestimmung eines Analyten in einer flüssigen Testprobe, wobei man:

a) eine erste Substanz an eine unlösliche, poröse Trägerplatte bindet, wobei die erste Substanz ausgewählt ist unter den, den Analyten bindenden Liganden und Rezeptoren, wobei der Träger von einer Schicht bedeckt ist aus wasserundurchlässigem Material mit einer darin ausgebildeten Öffnung und/oder mit einer unmittelbar darunter angeordneten Fläche aus hydrophilem Material, welches die transversale Diffusion der Flüssigkeit durch den Träger erhöht;

b) die Testprobe auf den unlöslichen Träger über die Öffnung, wenn die Deckschicht vorliegt, aufträgt;

c) die Testprobe durch den unlöslichen Träger, der die erste Substanz trägt, diffundieren läßt;

d) eine markierte zweite Substanz auf den unlöslichen Träger vor, zusammen mit oder nach dem Auftragen des Analyten aufträgt, wobei die zweite Substanz ausgewählt ist unter Liganden und Rezeptoren, welche durch Bindung an die erste Substanz oder an den Analyten immobilisierbar sind und mit kolloidalem Gold oder kolloidalem Silber markiert sind; und

e) die Präsenz und/oder Menge der immobilisierten, markierten zweiten Substanz bestimmt.

**2.** Verfahren nach Anspruch 1, worin für eine qualitative Bestimmung die Markierung in einer Menge vorliegt, die ausreicht, um ein deutliches Signal zu erzeugen, welches mit dem bloßen Auge erkenntlich ist, wenn die zweite Substanz in einer Menge oberhalb einer vorbestimmten Nachweisgrenze vorliegt, und das für das bloße Auge nicht erkenntlich ist, wenn die zweite Substanz in einer Menge unterhalb der vorbestimmten Nachweisgrenze vorliegt.

**3.** Verfahren nach Anspruch 1, worin die hydrophile Fläche fehlt und man zur quantitativen Bestimmung des Analyten die Testprobe lateral durch den unlöslichen Träger diffundieren läßt, um ein radiales Diffusionsmuster zu erhalten, und man den Durchmesser des Diffusionsmusters bestimmt.

**4.** Verfahren nach Anspruch 1 oder Anspruch 2, worin die Deckschicht aus wasserundurchlässigem Material vorliegt und die Diffusion der Testprobe transversal durch den unlöslichen Träger durch Anwenden eines negativen Drucks oder eines positiven Drucks oder durch Anbringen des hydrophilen Materials auf der der Öffnung gegenüberliegenden Seite des unlöslichen Trägers, erhöht.

**5.** Verfahren nach einem der Ansprüche 1 bis 4, wobei die Testprobe eine unbekannte Konzentration des Analyten und eine bekannte Konzentration der markierten zweiten Substanz enthält, wobei die zweite Substanz zur Bindung an die immobilisierte erste Substanz befähigt ist, wodurch ein kompetitiver Bindungstest bewirkt wird.

**6.** Verfahren nach einem der Ansprüche 1 bis 4, in dem die markierte zweite Substanz zur Bindung an den Analyten befähigt ist und nach dem Analyten auf den Träger aufgetragen wird.

**7.** Verfahren nach einem der Ansprüche 1 bis 6, worin die zweite Substanz mit Hilfe einer weiteren markierten Verbindung markiert wird, wobei diese Verbindung zur Gruppe von Liganden und Rezeptoren gehört, welche an die zweite Substanz binden.

**8.** Verfahren nach einem der Ansprüche 1 bis 6, wobei der Durchmesser der Öffnung bis zu etwa 5 mm beträgt.

**9.** Verfahren nach einem der Ansprüche 1 bis 7, worin die erste Substanz direkt oder indirekt, kovalent oder nicht-kovalent an den unlöslichen Träger gebunden ist.

**10.** Verfahren nach einem der Ansprüche 1 bis 8, worin die Liganden und Rezeptoren ausgewählt sind unter spezifisch wechselwirkenden Antigenen und Antikörpern, Zuckern und Lectinen, Nukleinsäuren, Enzymen und Substraten, Enzymen und Inhibitoren, Biotin und Avidin oder Komplexen davon, oder Immunglobulin und Staphylococcus Protein A oder Komplexen davon.

**11.** Verfahren nach einem der Ansprüche 1 bis 9, worin der unlösliche Träger ein polymeres Material ist, welches funktionelle Gruppen trägt, die an Proteine, Kohlehydrate oder Nukleinsäuren gebunden werden können.

**12.** Kit für die qualitatitve oder quantitative Bestimmung von Analyten in einer Testprobe, wobei der Kit umfaßt:

a) eine unlösliche, poröse Trägerplatte, an die eine erste Substanz gebunden ist, die ausgewählt ist unter für den Analyten spezifischen Liganden und Rezeptoren, wobei der Träger von einer Schicht aus wasserundurchlässigem Material bedeckt ist, in der eine Öffnung ausgebildet ist und/oder die unmittelbar darunter eine Fläche aus hydrophilem Material aufweist, welche die transversale Diffusion der Flüssigkeit durch den Träger erhöht, und

b) eine Lösung einer zweiten Substanz, wobei die zweite Substanz ausgewählt ist unter Liganden und Rezeptoren, welche die erste Substanz oder den Analyten binden, wobei die zweite Lösung eine bekannte Konzentration der zweiten Substanz umfaßt, welche mit einer Markierung, umfassend kolloidales Gold oder kolloidales Silber, konjugiert ist.

**13.** Kit nach Anspruch 11, worin bei Vorliegen der Deckschicht der Durchmesser der Öffnung bis zu 5 mm beträgt.

**14.** Kit nach Anspruch 11 oder Anspruch 12, worin die erste Substanz direkt oder indirekt, kovalent oder nicht-kovalent an den unlöslichen Träger gebunden ist.

**15.** Kit nach einem der Ansprüche 11 bis 13, worin die Liganden und Rezeptoren ausgewählt sind unter spezifisch wechselwirkenden Antigenen und Antikörpern, Zuckern und Lectinen, Nukleinsäuren, Enzymen und Substraten, Enzymen und Inhibitoren, Biotin und Avidin oder Komplexen davon, oder Immunglobulin und Staphylococcus Protein A oder Komplexen davon.

**Revendications**

**1.** Procédé pour la détermination quantitative ou qualitative d'un analyte dans un échantillon d'essai liquide, caractérisé en ce qu'il comprend les étapes consistant à :

(a) lier une première substance à un support poreux, en forme de feuille, insoluble, cette première substance étant choisie dans le groupe formé de ligands et de recepteurs liant ou fixant l'analyte précité, le support étant recouvert d'une couche de matière imperméable à l'eau, qui comprend un trou et/ou qui possède de manière adjacente sous-elle une feuille de matière hydrophile servant à augmenter la diffusion transversale de liquide à travers le support précité;

(b) appliquer l'échantillon à tester au support insoluble précité, à travers ledit trou, où ladite couche de couverture est présente;

(c) permettre à l'échantillon à tester de diffuser à travers le support insoluble précité portant ladite première substance;

(d) appliquer une seconde substance marquée au support insoluble précité avant, au moment de, ou après l'application dudit analyte, cette seconde substance étant choisie dans le groupe formé de ligands et de récepteurs capables d'être immobilisés par liaison à la première substance précitée ou audit analyte et étant marquée par de l'argent colloïdal ou de l'or colloïdal; et

(e) déterminer la présence et/ou la quantité de ladite seconde substance marquée et immobilisée.

**2.** Procédé suivant la revendication 1, caractérisée en ce qu'en vue de la détermination qualitative, ladite marque est présente en une proportion qui suffit à engendrer un signal clair qui est visible à l'oeil nu lorsque ladite seconde substance est présente en une proportion supérieure à la limite de détection prédeterminée et qui n'est pas visible à l'oeil nu lorsque ladite seconde substance est présente en une proportion inférieure à ladite limite de détection prédéterminée.

**3.** Procédé suivant la revendication 1, caractérisé en ce que ladite feuille hydrophile est absente et, en

EP 0 207 152 B1

vue de la détermination quantitative, on laisse l'analyte dans l'échantillon à tester diffuser latéralement à travers ledit support insoluble pour engendrer une plage de diffusion radiale et on mesure le diamètre de ladite plage de diffusion.

**4.** Procédé suivant la revendication 1 ou la revendication 2, caractérisé en ce que ladite couche de couverture en matière imperméable à l'eau est présente et la diffusion de l'échantillon à tester transversalement à travers le support insoluble précité est augmentée en utilisant une pression négative ou une pression positive ou en plaçant ladite matière hydrophile en position du côté dudit support insoluble opposé au trou précité.

**5.** Procédé suivant l'une quelconque des revendications 1 à 4, caractérisé en ce que l'échantillon à tester contient une concentration inconnue de l'analyte et une concentration connue de ladite seconde substance marquée, cette seconde substance étant capable de se lier à ladite première substance immobilisée, de manière à réaliser un titrage par liaison compétitive.

**6.** Procédé suivant l'une quelconque des revendications 1 à 4, caractérisé en ce que ladite seconde substance marquée est capable de se lier à l'analyte précité et on l'applique au support après l'analyte en question.

**7.** Procédé suivant l'une quelconque des revendications 1 à 6, caractérisé en ce que ladite seconde substance est marquée à l'aide d'un autre composé marqué, ce composé marqué appartenant au groupe formé de ligants et de récepteurs liant ladite seconde substance.

**8.** Procédé suivant l'une quelconque des revendications 1 à 6, caractérisé en ce que le diamètre du trou précité s'élève jusqu'à approximativement 5 mm.

**9.** Procédé suivant l'une quelconque des revendications 1 à 7, caractérisé en ce que ladite première substance est liée directement ou indirectement, de manière covalente ou non covalente, au support insoluble précité.

**10.** Procédé suivant l'une quelconque des revendications 1 à 8, caractérisé en ce que l'on choisit les ligands et les récepteurs dans les groupes formés d'anticorps et d'antigènes. des sucres et des lectines, des acides nucléiques, des enzymes et des substrats, des enzymes et des inhibiteurs, la biotine et l'avidine ou leurs complexes, ou l'immunoglobuline et la protéine A staphylocoxique, ou leurs complexes,qui interréagissent de manière spécifique.

**11.** Procédé suivant l'une quelconque des revendications 1 à 9, caractérisé en ce que le support insoluble est une matière polymérique qui porte des radicaux fonctionnels capables de se lier à des protéines, des hydrates de carbone ou des acides nucléiques.

**12.** Trousse pour la détermination qualitative ou quantitative d'un analyte dans un échantillon à tester caractérisée en ce qu'elle comprend :
(a) un support poreux, en forme de feuille, insoluble, qui est lié à une première substance choisie dans le groupe formé de ligands et de récepteurs, spécifiques dudit analyte, le support étant recouvert d'une couche de matière imperméable à l'eau, qui comporte un trou et/ou qui possède de manière adjacente en dessous d'elle une feuille de matière hydrophile servant à augmenter la diffusion transversale de liquide à travers le support précité et
(b) une solution d'une seconde substance, cette seconde substance étant choisie dans le groupe formé de ligants et de récepteurs se liant à ladite première substance ou audit analyte, ladite seconde solution comprenant une concentration connue de la seconde substance, conjuguée à une marque comprenant de l'argent colloïdal ou de l'or colloïdal.

**13.** Trousse suivant la revendication 11, caractérisée en ce que, lorsque ladite couche de couverture est présente, le diamètre du trou précité s'élève jusqu'à 5 mm.

**14.** Trousse suivant la revendication 11 ou la revendication 12, caractérisée en ce que ladite première substance est liée directement ou indirectement de manière covalente ou non covalente, au support insoluble précité.

20

**15.** Trousse suivant l'une quelconque des revendications 11 à 13, caractérisée en ce que l'on choisit les ligands et les récepteurs dans les groupes formés d'anticorps et d'antigènes,

des sucres et des lectines, des acides nucléiques, des enzymes et des substrats, des enzymes et des inhibiteurs, la biotine et l'avidine, ou leurs complexes ou l'immunoglobuline et la protéine A staphylocoxique, ou leurs complexes, qui interréagissent de manière spécifique.

FIG. Ia.

FIG. Ib.

FIG. Ic.

FIG. 2a.

FIG. 2b.

FIG. 2c.

## FIG. 3.

INACTIVATED PEROXIDASE

AREA OF
PATTERN IN
SQUARE mm

## FIG. 4.

GENTAMICIN

AREA OF
PATTERN IN
SQUARE mm

## *FIG. 5.*

THEOPHYLLINE

AREA OF
PATTERN IN
SQUARE mm

(y-axis: 0, 10, 20, 30, 40, 50, 60, 70, 80)
(x-axis: 0, 1.6, 3.2, 6.4, 12.8, 25.6)

## *FIG. 6.*

IMMUNOGLOBULIN

AREA OF
PATTERN IN
SQUARE mm

(y-axis: 0, 10, 20, 30, 40, 50, 60, 70, 80)
(x-axis: 0, 32, 64, 128, 256, 512)